# EUROPEAN PATENT SPECIFICATION

(11) **EP 0 887 081 B1**
(45) Date of publication and mention of the grant of the patent: **23.04.2003**
(21) Application number: 98304189.8
(22) Date of filing: 27.05.1998
(51) Int. Cl.: A61K 38/45, A61K 48/00, G01N 33/50, A61P 43/00

(54) **Human serum glucocorticoid regulated kinase, a target for chronic renal disease and diabetic nephropathy**
Humäne Serumglucocorticoidregulierte Kinase, ein Ziel für chronischer Nierenerkrankung und diabetische Nephropathie
Kinase régulée par les glucocorticoides du sérum humain, une cible pour maladies rénales chroniques et néphropaties diabétiques

(30) Priority: 27.06.1997 US 51124 P
(43) Date of publication of application: 30.12.1998
(73) Proprietor: SMITHKLINE BEECHAM CORPORATION, Philadelphia Pennsylvania 19103 (US)
(72) Inventor: Kumar, Janet M., King of Prussia, Pennsylvania 19406 (US)
(74) Representative: Stott, Michael John

(56) References cited:
- EP-A- 0 861 896
- WO-A-95/02823
- WO-A-98/11234
- DE-A- 19 813 839
- S WALDEGGER ET AL: "Cloning and characterization of a putative human serine/threonine protein kinase transcriptionally modified during anisotonic and isotonic alterations of cell volume" PROCEEDINGS OF THE NATIONAL ACADEMY OF SCIENCES OF USA,US,NATIONAL ACADEMY OF SCIENCE. WASHINGTON, vol. 94, April 1997, pages 4440-4445, XP002079929
- Webster et al.: 'Characterisation of sgk, a novel member of the serine/threonine protein kinase gene family which is transcriptionally induced by glucocorticoids and serum'. Mol. Cell. Biol. (1993 April) 13(4): 2031-40 (cited in the application)
- Maiyar et al.: 'Repression of glucocorticoid receptor transactivation and DNA binding of a glucocorticoid response element within the serum / glucocorticoid-inducible protein kinase (sgk) gene promoter by the p53 tumor suppressor protein'. Mol. Endocrinol. (1997 March) 11(3):312-29

## Description

### FIELD OF INVENTION

The field of the invention is treatment of chronic renal failure and diabetic nephropathy.

### BACKGROUND OF THE INVENTION

The serum glucocorticoid regulated kinase (*sgk*) was discovered in a rat mammary tumor cell line (Con8.hd6) to be a novel member of the serine/threonine protein kinase family whose expression is transcriptionally regulated by glucocorticoids and serum (Webster et al., 1993, Mol. Cell Biol. 13:2031-2041). It is expressed in other rat tissues such as the ovary, thymus and lung with lower levels in most other tissues (Webster et al., 1993, Mol. Cell Biol. 13:2031-2041). Sequence analysis of the full length rat *sgk* mRNA predicts a 431 amino acid protein of 49 kDa. The *sgk* protein contains a putative 270 amino acid catalytic domain made up of 11 distinct subdomains, one of which suggests phosphorylation of serine/threonine substrates. *Sgk* shares sequence homology with other kinases in this region. These include the human rac a, rat protein kinase C-b, rat ribosomal protein S6 kinase an the mouse cyclic AMP dependent protein kinase. Despite the homology with other kinases, *sgk* has yet to be shown to be a functional kinase.

The promoter region of the rat *sgk* gene contains a functional glucocorticoid responsive element (GRE) and the induction of *sgk* appears to be a glucocorticoid receptor-specific response (Webster et al., 1993, Mol. Cell Biol. 13:2031-2041). A similar increase in *sgk* transcript levels is observed in rat mammary tumor cells after treatment with either dexamethisone, hydrocortisone or corticosterone all of which use the glucocorticoid receptor pathway whereas steroids which have an alternate means of gene activation such as cholesterol, progesterone, b-estradiol, and testosterone were unable to induce *sgk* mRNA expression(Webster et al., 1993, Mol. Cell Biol. 13:2031-2041). Serum also stimulates *sgk* mRNA expression in these cells although the pathway of activation is unclear. Recently, p53, a tumor supressor protein, has been found to repress *sgk* promoter activity (Maiyar et al., 1997, Mol. Endocrin. 11:312-329). This represents the first example of a hormone regulated kinase gene regulated by p53 (Maiyar et al., 1996, J. Biol. Chem. 271:12414-12422).

The function of *sgk* in the cell is unclear. In some cell types *sgk* may play a role in cell growth. In the Con8.hd6 cells, glucocorticoid treatment, which increases *sgk* expression, decreases the proliferation. However, in other cell lines dexamethisone increases *sgk* levels without affecting cell growth. Some data suggests that it may play a role in mitogenic response in the G₀-G₁ transition in quiescent Rat2 fibroblasts since it is induced in response to serum stimulation and has a rapid half life like other immediate-early response genes (Webster et al., 1993, J. Biol. Chem. 268:11482-11485). One group proposes that *sgk* is involved in wound healing and regeneration in the central nervous system. *Sgk* was found to be strongly expressed in glial cells and oligodendrocytes surrounding lesions in the injured rat brain (Imaizumi et al., 1994, Mol. Brain Res. 26:189-196). Recently, sgk transcript levels were found to be influenced by alterations in anisotonic and isotonic conditions indicating that it may play a role in cellular response to cell volume (Waldegger et al., 1997, Proc. Natl. Acad. Sci.. 94:4440-4445). Whatever its role, *sgk* is most likely involved in a complex series of phosphorlyations/dephosphorlyations regulated separately and/or concurrently by a number of extracellular and intracellular factors such as glucocorticoids, serum, growth factors and p53.

A number of animal model systems have been studied to provide clues as to the cause(s) of renal failure. Molecules whose expression is either increased or decreased in the diseased kidney of these model systems may be important mediators of kidney failure. Differential display PCR was performed to identify such genes in the kidneys of lean and obese db/db mice. One of the genes increased in obese mouse kidney was found to be a 500 base pair cDNA 91% identical with the rat *sgk.* Northern analysis demonstrated that *sgk* mRNA levels were increased 5 fold in the kidney of diabetic obese mice as compared to lean littermates. Of the other tissues examined, heart, brain and liver, *sgk* mRNA levels were similar between lean and obese mice. Thus, *sgk* induction is kidney specific. Further analysis indicated that *sgk* mRNA expression was also increased 2 fold in the kidney of diabetic humans as compared to control kidneys. This is the first demonstration that *sgk* may be involved in a diseased state, namely renal disease.

There remains a need for compositions for treatment of chronic renal disease which serve to diminish or ablate disease leading to renal failure and either death or dependence on dialysis.

### SUMMARY OF THE INVENTION

The invention relates to the manufacture of medicaments for use in the treatment of renal failure or diabetic nephropathy.

### DESCRIPTION OF THE INVENTION

### Definitions

The following definitions are provided to facilitate understanding of certain terms used frequently herein.

"Human *sgk*"or "human sgk polypeptide" refers, among others, generally to a polypeptide having the amino acid sequence set forth in SEQ ID NO:2 or an allelic variant thereof.

"Human *sgk* activity or human *sgk* polypeptide activity" or "biological activity of the human *sgk* or human *sgk* polypeptide" refers to the metabolic or physiologic function of said human *sgk* including similar activities or improved activities or these activities with decreased undesirable side-effects. Also included are antigenic and immunogenic activities of said human *sgk.*

"Human *sgk* gene" refers to a polynucleotide having the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single- stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al*., "Analysis for protein modifications and nonprotein cofactors", *Meth Enzymol* (1990) 182:626-646 and Rattan *et al*., "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: (COMPUTATIONAL MOLECULAR BIOLOGY, Lesk, A.M., ed., Oxford University Press, New York, 1988; BIOCOMPUTING: INFORMATICS AND GENOME PROJECTS, Smith, D.W., ed., Academic Press, New York, 1993; COMPUTER ANALYSIS OF SEQUENCE DATA, PART I, Griffin, A.M., and Griffin, H.G., eds., Humana Press, New Jersey, 1994; SEQUENCE ANALYSIS IN MOLECULAR BIOLOGY, von Heinje, G., Academic Press, 1987; and SEQUENCE ANALYSIS PRIMER, Gribskov, M. and Devereux, J., eds., M Stockton Press, New York, 1991). While there exist a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCS program package (Devereux, J., *et al., Nucleic Acids Research* (1984) 12(1):387), BLASTP, BLASTN, FASTA (Atschul, S.F. *et al., J Molec Biol* (1990) 215:403).

"Chronic renal failure" is the progressive loss of functional renal mass, accompanied by compensatory growth and remodeling. The molecular and cellular events that take place during chronic renal failure include release of growth factors, proliferation of glomerular mesangial cells and expansion of extracellular matrix (Klahr et al., 1988, New Engl. J. Med 318:1657-1666; Striker et al., 1989, In: Klahr, (Ed.) Seminars in Nephrology, pp. 318, Philadelphia, (W.B. Saunders); Ebihara et al., 1993, J. Am. Soc. Nephrol. 3:1387-1397).

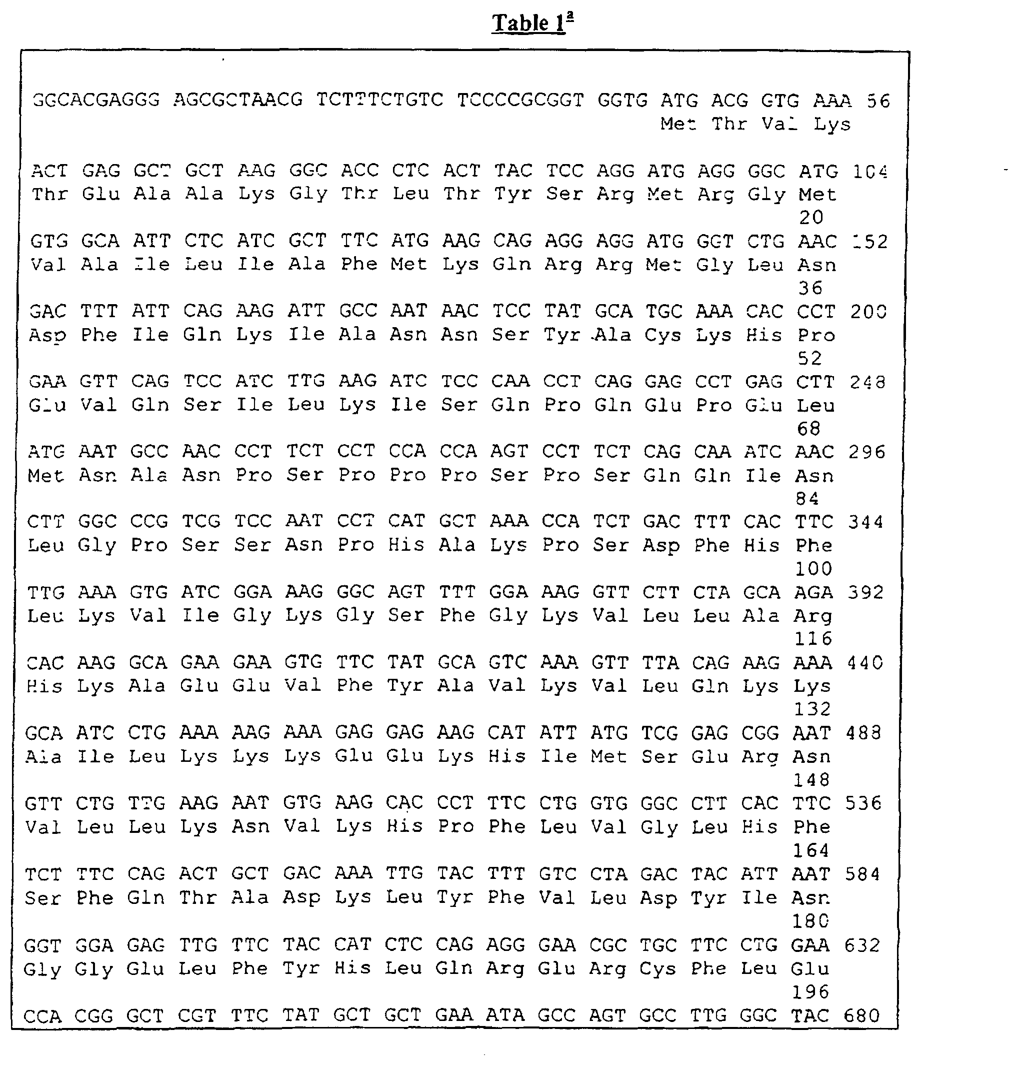

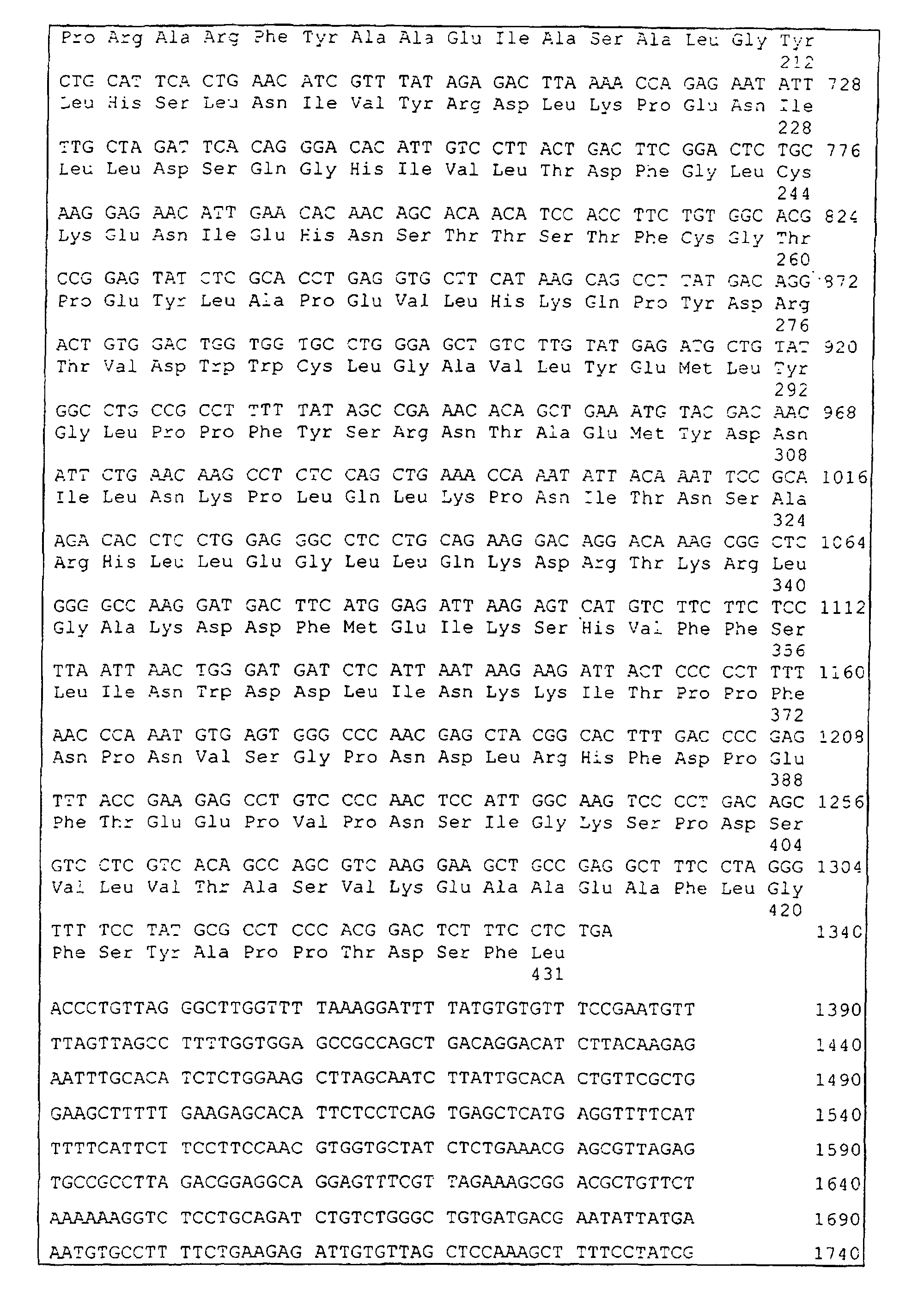

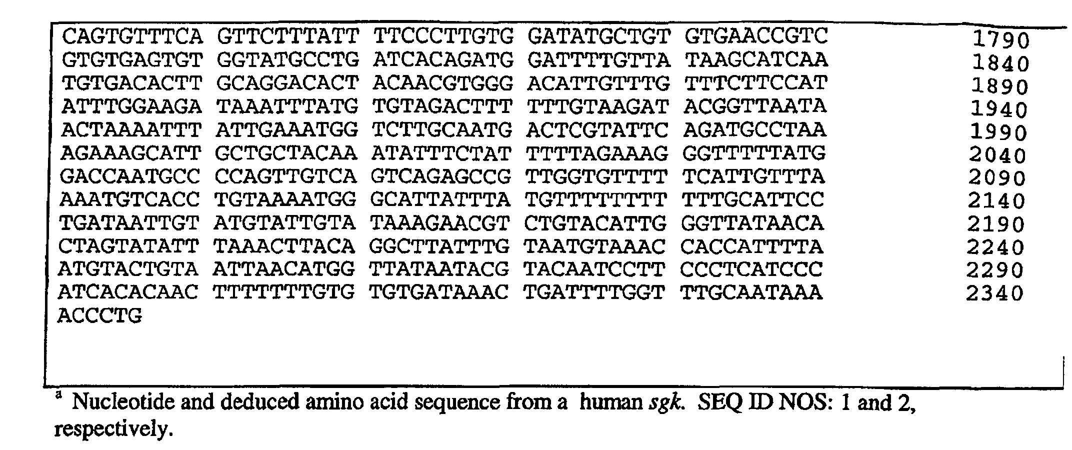

### Screening Assays

Human sgk polypeptides may be employed in screening process for compounds which activate (agonists) or inhibit activation of (antagonists, or otherwise called inhibitors) human sgk polypeptide. Thus, human sgk polypeptide may also be used to assess identify antagonists from, for example, cells, cell-free preparations, chemical libraries, and natural product mixtures. These antagonists may be natural substrates, ligands, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

In general, such screening procedures may involve using appropriate cells which express the human sgk polypeptide or respond to human sgk polypeptide. Such cells include cells from mammals, yeast, *Drosophila* or *E. coli.* Cells which express the human sgk polypeptide (or cell membrane containing the expressed polypeptide) or respond to human sgk polypeptide are then contacted with a test compound to observe binding, or stimulation or inhibition of a functional response. The ability of the cells which were contacted with the candidate compounds is compared with the same cells which were not contacted for human sgk activity.

The assays may simply test binding of a candidate compound wherein adherence to the cells bearing the human sgk polypeptide is detected by means of a label directly or indirectly associated with the candidate compound or in an assay involving competition with a labeled competitor. Further, these assays may test whether the candidate compound results in a signal generated by activation of the human sgk polypeptide, using detection systems appropriate to the cells bearing the human sgk polypeptide. Inhibitors of activation are generally assayed in the presence of a known agonist and the effect on activation by the agonist by the presence of the candidate compound is observed.

The human sgk cDNA, protein and antibodies to the protein may also be used to configure assays for detecting the effect of added compounds on the production of human sgk mRNA and protein in cells. For example, an ELISA may be constructed for measuring secreted or cell associated levels of human sgk polypeptide using monoclonal and polyclonal antibodies by standard methods known in the art, and this can be used to discover agents which may inhibit or enhance the production of human sgk (also called antagonist or agonist, respectively) from suitably manipulated cells or tissues.

The human sgk polypeptide may be used to identify membrane bound or soluble receptors, if any, through standard receptor binding techniques known in the art. These include, but are not limited to, ligand binding and crosslinking assays in which the human sgk is labeled with a radioactive isotope (eg 125I), chemically modified (eg biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of human sgk which compete with the binding of human sgk to its receptors, if any. Standard methods for conducting screening assays are well understood in the art.

Examples of potential human sgk polypeptide antagonists include peptides comprising specific portions of human sgk; peptidomimetics having anti-sgk activity; antibodies; or in some cases, oligonucleotides or proteins which are closely related to the ligands, substrates, receptors, etc., as the case may be, of the human sgk polypeptide, e.g., a fragment of the ligands, substrates, receptors; or small chemical molecules which bind to the polypetide of the present invention but do not elicit a response, so that the activity of the polypeptide is prevented.

The human sgk can be made by a variety of recombinant genetic engeering techniques. The isolated nucleic acids, particularly the DNAs can be introduced into expression vectors by operativley linking the DNA to the necessary expression control regions (e.g. regulatory regions) required for gene expression. The vectors can be introduced into the appropriate host cells such as prokaryotic (e.g. bacterial), or eukaryotic (e.g. yeast or mammalian) cells by methods well known in the art (Ausubel et al., supra). The coding sequences for the desired proteins having been prepared or isolated, can be cloned into any suitable vector or replicon. Numerous cloning vectors are know to those of skill in the art, and the selection of an appropriate cloning vector is a matter of choice. The gene can be placed under the control of a promoter, ribosome binding site (for bacterial expression) and optionally, an operator (collectively referred to herein as "control" elements), so that the DNA sequence encoding the desired protein is transcribed into RNA in the host cell transformed by a vector containing this expression construction. The coding sequence may or may not contain a signal peptide or leader sequence. The subunit antigens of the present invention can be expressed using, for example, the E. coli tac promoter or the protein A gene (spa) promoter and signal sequence. Leader sequences can be removed by the bacterial host in post-translational processing. See, e.g., U.S. Patent Nos. 4,431,739; 4,425,437;4,338,397.

In additon to control sequences, it may be desirable to add regulatory sequences with allow for regulation of the expression of the protein sequences relative to the growth of the host cell. Regulatory sequences are known to those of skill in the art, and examples include those which cause the expression of a gene to be turned on or off in response to chemical or physical stimulus, including the presence of a regulatory compound. Other types of regulatory elements may also be present in the vector, for example, enhancer sequences.

An expression vector is constructed so that the particular coding sequence is located in the vector with the appropriate regulatory sequences, the positioning and orientation of the coding sequences with respect to the control sequences being such that the coding sequence is transcribed under the "control" of the control sequences (i.e. RNA polymerase which binds to the DNA molecule at the control scquences transcribes the coding sequence). Modification of the sequences encoding the particular protein of interest may be desirable to achieve this end. For example, in some cases it may be necessary to modify the sequence so that it may be attached to the control sequences with the appropriate orientation; i.e., to maintain the reading frame. The control sequences and other regulatory sequences may be ligated to the coding sequence prior to insertion into a cloning vector. Alternatively, the coding sequence can be cloned directly into an expression vector which already contains the control sequences and an appropriate restriction site.

In some cases, it may be desirable to add sequences which cause the secretion of the polypeptide from the host organism, with subsequent cleavage of the secretory signal. Alternatively, gene fusions may be created whereby the gene encoding the binding protein of interest is fused to a gene encoding a protein with other desirable properties. For example, a fusion partner could provide known assayable activity (e.g. enzymatic) which could be used as an alternative means of selecting the binding protein. The fusion partner could be a structural element, such as a cell surface element such that the binding protein (a normally cytosolic component) could be displayed on the cell surface in the form of a fusion protein. The fusion protein (e.g., fused to a FLAG peptide) is engineered to allow for purification of the expressed protein after host cell expression. It may also be desirable to produce mutants or analogs of the protein of interest. Mutants or analogs may be prepared by the deletion of a portion of the sequence encoding the protein, by insertion of a sequence, and/or by substitution of one or more nucleotides within the sequence. Techniques for modifying nucleotide sequences, such as site directed mutagenesis and the formation of fusion proteins, are well known to those skilled in the art. See, e.g. T. Maniatis et al., supra; DNA Cloning, Vols. I and II, supra; Nucleic Acid Hybridization, supra.

A number of prokaryotic expression vectors are known in the art. See, e.g., U.S. Patent Nos.4,578,355:4,440,859;4,436,815,4,431,740;4,431,739;4,428,941;4,425,437,4,418,149; 4,411,994; 4,366,246;4,342,832; see also U.K. Patent Applications GB 2,121,054; GB 2,008,123; GB 2,007,675; and European Patent Application 103,395. Yeast expression vectors are also known in the art. See, e.g., U.S. Patent Nos. 4,446,235; 4,443,539; 4,430,428; see also European Patent Applications 103,409; 100,561;96,491. pSV2neo (as described in J. Mol. Appl. Genet. 1;327-341) which uses the SV40 late promoter to drive expression in mammalian cells or pCDNAlneo, a vector derived from pcDNA1 (Mol. Cell Biol.7:4125-29) which uses the CMV promoter to drive expression. Both these latter two vectors can be employed for transient or stable (e.g. using G418 or hygromycin resistance) expression in mammalian cells. Insect cell expression systems, e.g., Drosophila, are also useful, see for example, PCT applications US 89/05155 and US 91/06838 as well as EP applications 88/304093.3 and Baculovirus expression systems.

Depending on the expression system and host selected, the proteins of the present invention are produced by growing host cells transformed by an expression vector described above under conditions whereby the protein of interest is expressed. The protein is then isolated from the host cells and purified. If the expression system secretes the protein into growth media, the protein can be purified directly from the media. If the protein is not secreted, it is isolated from cell lysates or recovered from the cell membrane fraction. The selection of the appropriate growth conditions and recovery methods are within the skill of the art.

The knowledge that the human sgk may encode a protein kinase suggests that recombinant forms, described above, can be used to establish a protein kinase activity. Typically this would involve the direct incubation of purified human sgk with a protein or peptide substrate in the presence of γ-32P- ATP, followed by the measurement of radioactivity incorporated into the substrate by separation and counting. Separation methods include immunoprecipitation, conjugation of substrate to a bead allowing separation by centrifugation or determination of incorporation by scintillation proximity assay, SDS-PAGE followed by autoradiography or biosensor analysis. While the specific substrates are not yet known, candidates include human sgk itself (autophosphorylation), myelin basic protein, casein, histone and HSP27. Other substances might be discovered by incubating human sgk with random peptides conjugated to solid supports or displayed on the surface of phage or by incubation of human sgk with mammalian cell lysates and γ-32P- ATP, followed by separation of the labelled target proteins, and sequencing. The protein kinase activity of human sgk may require incubation with a specific upstream effector. This may be achieved by preincubating human sgk with lysates from a variety of stimulated eukaryotic cells and ATP.

Without wishing to be bound by any particular theory of the functioning of the human sgk of this invention, it is believed that among the useful inhibitors of human sgk function are those compounds which inhibit the kinase activity of the human sgk. Other sites of inhibition are, of course, possible owing to its position in a signal transduction cascade. Therefore, inhibiting the interaction of human sgk with one or more of its upstream or downstream modulators/substrates is also contemplated by this invention. Inhibitors of protein-protein interactions between human sgk
and other factors could lead to the development of pharmaceutical agents for the modulation of human sgk activity.

In one specific embodiment, a method to identify possible inhibitors of the *sgk* is described here. The peptide substrate of *sgk* will be biotinlyated at one end. It will then be added to a 96 well streptavidin coated flash plate. The high affinity binding of the biotin to streptavidin in the wells will anchor the peptide in the well and allow the residues to be accessible for phosphorylation. Purified active *sgk*, ³³P-gamma-ATP and compounds will then be incubated with the peptide at a suitable temperature for kinase activity to occur. The plates will be washed and the amount of phosphorlyated peptide will be determined. Those wells with the least amount of radioactivity could contain a possible inhibitor.

### Human sgk binding molecules and assays

Human sgk could be used to isolate proteins which interact with it and this interaction could be a target for interference. Inhibitors of protein-protein interactions between human sgk and other factors could lead to the development of pharmaceutical agents for the modulation of human sgk activity. As used herein, the term "modulate" refer to affecting the human sgk function.

Genes encoding proteins for binding molecules to human sgk can be identified by numerous methods known to those of skill in the art, for example, ligand panning and FACS sorting. Such methods are described in many laboratory manuals such as, for instance, Coligan et al., Current Protocols in Immunology 1 (Rivett, A.J. Biochem. J. 291:1-10 (1993)): Chapter 5 (1991).

For example, the yeast two-hybrid system provides methods for detecting the interaction between a first test protein and a second test protein, in vivo, using reconstitution of the activity of a transcriptional activator. The method is disclosed in U.S. Patent No. 5,283,173; reagents are available from Clontech and Stratagene. Briefly, human sgk cDNA is fused to a Gal4 transcription factor DNA binding domain and expressed in yeast cells. cDNA library members obtained from cells of interest are fused to a transactivation domain of Gal4. cDNA clones which express proteins which can interact with human sgk will lead to reconstitution of Gal4 activity and transactivation of expression of a reporter gene such as Gal1-lacZ. The sgk cDNA which is fused to the Gal4 transcription factor DNA binding domain may be mutated in one or more amino acids, method of which is described above, to ehance interaction of kinase with substrate.

An alternative method is screening of λgt11, λZAP (Stratagene) or equivalent cDNA expression libraries with recombinant human sgk. Recombinant human sgk protein or fragments thereof are fused to small peptide tags such as FLAG, HSV or GST. The peptide tags can possess convenient phosphorylation sites for a kinase such as heart muscle creatine kinase or they can be biotinylated. Recombinant human sgk can be phosphorylated with 32[P] or used unlabeled and detected with streptavidin or antibodies against the tags. λgt11cDNA expression libraries are made from cells of interest and are incubated with the recombinant human sgk, washed and cDNA clones isolated which interact with human sgk. See, e.g., T. Maniatis et al, supra.

Another method is the screening of a mammalian expression library in which the cDNAs are cloned into a vector between a mammalian promoter and polyadenylation site and transiently transfected in COS or 293 cells followed by detection of the binding protein 48 hours later by incubation of fixed and washed cells with a labelled human sgk, preferably iodinated, and detection of bound human sgk by autoradiography. See Sims et al., Science 241:585-589 (1988) and McMahan et al., EMBO J. 10:2821-2832 (1991). In this manner, pools of cDNAs containing the cDNA encoding the binding protein of interest can be selected and the cDNA of interest can be isolated by further subdivision of each pool followed by cycles of transient transfection, binding and autoradiography. Alternatively, the cDNA of interest can be isolated by transfecting the entire cDNA library into mammalian cells and panning the cells on a dish containing human sgk bound to the plate. Cells which attach after washing are lysed and the plasmid DNA isolated, amplified in bacteria, and the cycle of transfection and panning repeated until a single cDNA clone is obtained. See Seed et al, Proc. Natl. Acad. Sci. USA 84:3365 (1987) and Aruffo et al., EMBO J. 6:3313 (1987). If the binding protein is secreted, its cDNA can be obtained by a similar pooling strategy once a binding or neutralizing assay has been established for assaying supernatants from transiently transfected cells. General methods for screening supernatants are disclosed in Wong et al., Science 228:810-815 (1985).

Another alternative method is isolation of proteins interacting with human sgk directly from cells. Fusion proteins of human sgk with GST or small peptide tags are made and immobilized on beads. Biosynthetically labeled or unlabeled protein extracts from the cells of interest are prepared, incubated with the beads and washed with buffer. Proteins interacting with human sgk are eluted specifically from the beads and analyzed by SDS-PAGE. Binding partner primary amino acid sequence data are obtained by microsequencing. Optionally, the cells can be treated with agents that induce a functional response such as tyrosine phosphorylation of cellular proteins. An example of such an agent would be a growth factor or cytokine such as interleukin-2.

Another alternative method is immunoaffinity purification. Recombinant human sgk is incubated with labeled or unlabeled cell extracts and immunoprecipitated with anti- human sgk antibodies. The immunoprecipitate is recovered with protein A-Sepharose and analyzed by SDS-PAGE. Unlabelled proteins are labeled by biotinylation and detected on SDS gels with streptavidin. Binding partner proteins are analyzed by microsequencing. Further, standard biochemical purification steps known to those skilled in the art may be used prior to microsequencing.

Yet another alternative method is screening of peptide libraries for binding partners. Recombinant tagged or labeled human sgk is used to select peptides from a peptide or phosphopeptide library which interact with human sgk. Sequencing of the peptides leads to identification of consensus peptide sequences which might be found in interacting proteins.

human sgk binding partners identified by any of these methods or other methods which would be known to those of ordinary skill in the art as well as those putative binding partners discussed above can be used in the above assay method. Assaying for the presence of human sgk/binding partner complex are accomplished by, for example, the yeast two-hybrid system, ELISA or immunoassays using antibodies specific for the complex. In the presence of test substances (i.e. inhibitors or antagonists) which interrupt or inhibit formation of human sgk/binding partner interaction, a decreased amount of complex will be determined relative to a control lacking the test substance.

Assays for free human sgk or binding partner are accomplished by, for example, ELISA or immunoassay using specific antibodies or by incubation of radiolabeled human sgk with cells or cell membranes followed by centrifugation or filter separation steps. In the presence of test substances which interrupt or inhibit formation of human sgk/binding partner interaction (i.e. inhibitors or antagonists), an increased amount of free human sgk or free binding partner will be determined relative to a control lacking the test substance.

Human sgk polypeptides also can be used to assess human sgk binding capacity of human sgk binding molecules in cells or in cell-free preparations.

### Formulation and Administration

Antagonists of human sgk polypeptides may be formulated in combination with a suitable pharmaceutical carrier. Such formulations comprise a therapeutically effective amount of the antagonist and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art.

Polypeptides and other compounds of the present invention may be employed alone or in conjunction with other compounds, such as therapeutic compounds.

Preferred forms of the pharmaceutical compositions include those adapted for administration by injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo*, and for example, by the use of a retroviral plasmid vector. The cells are then introduced into the subject.

### Examples

The examples below are carried out using standard techniques, which are well known and routine to those of skill in the art, except where otherwise described in detail. The examples illustrate, but do not limit the invention.

### Example 1

Sgk mRNA levels are increased in the kidney of a chronic renal failure mouse model. This is shown by a northern blot with 15 ug of total RNA extracted from the kidneys of lean and diabetic db/db mice and probed with a 500 base pair 32P labeled cDNA encoding the mouse sgk. The message specific for sgk is 2.4 kilobases (kb) and is induced 5-6 fold in the kidneys of the diabetic mice as compared to lean mice.

### Example 2

The increased expression of sgk in the kidney of the diabetic mice is specific for the kidney. This is indicated by a northern blot with 10 ug of total RNA extracted from the liver, brain, heart and kidney of lean and diabetic db/db mice. The 2.4 kb sgk message is expressed in the brain, heart and kidney but not in the liver of these animals. However, sgk mRNA is only induced in the kidneys of the diabetic animals as compared to the lean animals and not in the other tissues.

### Example 3

Not only is sgk message induced in a rodent renal failure model but its levels are increased in human diseased kidneys as well. A northern blot with 15 ug of total RNA extracted from the kidney of normal and diabetic patients was probed with a 500 base pair 32P labeled cDNA encoding the mouse sgk. The 2.4 kb sgk mRNA is induced 2 - 3 fold in the diabetic kidney as compared to the normal kidney.

## Claims

1. Use of:
(a) an antagonist to human sgk polypeptide; or
(b) a nucleic acid molecule that inhibits expression of the nucleotide sequence encoding human sgk polypeptide
in the manufacture of a medicament for use in the treatment of chronic renal failure or diabetic nephropathy.

## Patentansprüche

1. Verwendung
(a) eines Antagonisten zu dem menschlichen sgk-Polypeptid; oder
(b) eines Nucleinsäuremoleküls, das die Expression der das menschliche sgk-Polypeptid codierenden Nucleotidsequenz hemmt,
für die Herstellung eines Arzneimittels zur Verwendung bei der Behandlung von chronischem Nierenversagen oder diabetischer Neuropathie.

## Revendications

1. Utilisation :
(a) d'un antagoniste du polypeptide sgk humain ; ou
(b) d'une molécule d'acide nucléique qui inhibe l'expression de la séquence de nucléotides codant pour le polypeptide sgk humain
dans la production d'un médicament destiné à être utilisé dans le traitement de l'insuffisance rénale chronique ou de la néphropathie diabétique.
